# EUROPEAN PATENT SPECIFICATION

(11) **EP 2 572 748 B1**
(45) Date of publication and mention of the grant of the patent: **21.05.2014**
(21) Application number: 11182116.1
(22) Date of filing: 21.09.2011
(51) Int. Cl.: A61M 16/18

(54) **Apparatus and method for supplying breathing gas for subject breathing**
Vorrichtung und Verfahren zur Bereitstellung eines Atemgases zur Atemunterstützung eines Menschen
Appareil et procédé pour fournir du gaz respiratoire pour la respiration d'un sujet

(43) Date of publication of application: 27.03.2013
(73) Proprietor: General Electric Company, Schenectady, NY 12345 (US)
(72) Inventor: Heinonen, Erkki, 00750 Helsinki (FI); Kokkila, Johannes, 00200 Helsinki (FI)
(74) Representative: Illingworth-Law, William Illingworth

(56) References cited:
- EP-A1- 0 761 249
- EP-A2- 1 082 973
- WO-A1-2006/065202
- DE-C1- 10 105 434
- GB-A- 2 400 566
- US-A- 5 509 405
- US-A1- 2002 069 876
- US-A1- 2011 155 131

## Description

### BACKGROUND OF THE INVENTION

This disclosure relates generally to an apparatus and method for supplying breathing gas for subject breathing. The apparatus comprises an evaporation chamber for receiving and evaporating a liquid anesthetic agent and which evaporation chamber comprises an inlet opening for a fresh gas for mixing evaporated anesthetic agent with the fresh gas and which evaporation chamber also comprises an outlet opening for conducting the gas mixture including evaporated anesthetic agent out from the evaporation chamber. The disclosure also relates to an arrangement for providing an inspiration gas to lungs of a subject.

Presently, anesthesia machines are optimized for delivering anesthesia to a patient using volatile anesthetic agent liquids. In such systems, the anesthetic agent is evaporated and mixed into the fresh breathing gas stream in a controlled manner according to user dialed concentration to conform a gas mixture for anesthetizing the patient for a surgical operation. Presently known volatile anesthetic agents are halogenated hydrocarbon chains, such as halothane, enflurane, isoflurane, sevoflurane and desflurane. Of these three last are dominating in anesthesia practice that the use of the first ones is disappearing.

Since volatile anesthetic agents are expensive and environmentally damaging greenhouse gases, anesthesia machines have been developed to minimize the use of these gases. To keep patient anesthetized, a defined brain partial pressure for the anesthetic agent is required. This partial pressure is maintained by keeping the anesthetic agent partial pressure in the lungs adequate. During a steady state, the lung and body partial pressures are equal, and no net exchange of the anesthetic agent occurs between the blood and the lungs. However, to provide oxygen and eliminate carbon dioxide, continuous lung ventilation is required.

Anesthesia machines designed to deliver volatile anesthetic agents are designed to provide oxygen to the patient and eliminate carbon dioxide, while preserving the anesthetic gases. These goals are typically met using a re-breathing circuit, where an exhaled gas is reintroduced into the inhalation limb leading to the patient. In such a re-breathing circuit, carbon dioxide is absorbed from the expired gases by a carbon dioxide absorber. Before inhalation by the patient, the inhalation gas is supplied with additional oxygen and evaporated anesthetic agents based upon the therapy demand. The anesthetic agent is added to the fresh gas stream in an evaporator, which typically is an agent specific exchangeable add-on module to the anesthesia machine. In this arrangement, the additional fresh gas flow added to the re-breathing circuit can at minimum correspond to patient oxygen consumption, which can be as low as 200 mL/min whereas the patient ventilation may be 5-10 L/min. The positive pressure inspiration is typically carried out using a ventilator, which is typically gas driven. In these ventilators, the patient breathing gas is pressurized by controlling a ventilator drive gas flow through a separation system separating the breathing gas from the ventilator drive gas.

Anesthesia evaporators have relied on passive evaporation of the volatile liquid. For this purpose the evaporation chamber included a wick hanging on one end at the liquid. Capillary forces made the liquid rise up the wick to the gas phase above the liquid providing large surface area to equilibrate the surrounding gas with the agent vapor to the agent vapor pressure, which depends on the liquid temperature. On these types of evaporators the fresh gas flow is guided through the wick chamber to saturate with the vapor. The evaporator output concentration is controlled by adjusting flow division to paths going through- and bypassing the wick chamber. For this purpose the wick chamber gas outlet channel comprises an adjustable flow resistor whereas the gas inlet channel is open. The vapor pressure of the liquids provided a good basis for this when the boiling points of the liquids varied around 50°C. However, the era of desflurane having boiling point at sea level pressure close to normal room temperature required different approach.

The problem to be handled with desflurane evaporators is that at temperatures above room temperature the liquid reservoir creates spontaneous pressure. Using the traditional system this would mean uncontrolled agent delivery backwards through the wick chamber inlet channel. To solve this liquid chamber pressure is maintained by providing additional heat to the liquid. From the pressurized liquid chamber the evaporated agent flow is controlled with flow control system to adjust the evaporator output concentration. This control system includes also means to compensate for different fresh gas flows. Also controlled opening of the liquid chamber gas inlet channel has been used to let the liquid temperature vary around the boiling point. Such controlled opening needs knowledge of pressure balance between liquid chamber and the fresh gas line.

Today's anesthetic evaporators are dominantly mechanically controlled modules of anesthesia delivery system. Being mechanical they do not integrate modern operation room information system. Current trends however involve sophisticated communication between system components to facilitate automatic record keeping, system monitoring and advanced system features like control of anesthesia delivery from measured entity correlating with the anesthesia status of the patient. Such features require electronically controlled evaporator.

Using the traditional evaporation technologies on electronically controlled evaporators requires use of various sensors and actuators to provide information for accurate and safe operation of the system. The traditional liquid chamber is big when accommodating room for both liquid and gas phases increasing the whole evaporator size. Furthermore the liquid chamber accommodating also gas space involves a safety problem: In overfilling the liquid chamber such a way that it prevents gas flow may result liquid flow out from the system and agent overdosing. Therefore the evaporators must have means to prevent liquid flow. As a result of these current evaporators are complicated, large in size and expensive to manufacture.

WO 2006/055202 discloses an anesthetic liquid vaporizer comprising a vaporizing chamber receiving anesthetic liquid from an external liquid source via a liquid supplier which can be controlled. Liquid anesthetic is vaporized in the chamber when the liquid is exposed to a heated surface. Also a vaporizing method is disclosed.

GB 2400566 discloses an anesthetic vaporizer with a reservoir for liquid anesthetic agent, which reservoir can be pressurized and the liquid conveyed to a vaporizing device to vaporize the liquid for delivering together with a carrier gas to a patient. The pressurization and the conveyance of the liquid anesthetic agent to the vaporizing device are controlled independently of the carrier gas supply.

### BRIEF DESCRIPTION OF THE INVENTION

The above-mentioned shortcomings, disadvantages and problems are addressed herein which will be understood by reading and understanding the following specification.

In an embodiment an apparatus for supplying anesthetic agent to a fresh gas flow includes an evaporation chamber for receiving and evaporating a liquid anesthetic agent and which evaporation chamber includes an inlet opening for a fresh gas for mixing evaporated anesthetic agent with the fresh gas and which evaporation chamber also includes an outlet opening for conducting the gas mixture including evaporated anesthetic agent out from the evaporation chamber. The apparatus for supplying anesthetic agent to a fresh gas flow also includes a liquid chamber for a liquid anesthetic agent, and which liquid anesthetic agent is received by the evaporation chamber from the liquid chamber along an exit line, and a compressor applying a pressure to the chamber. The apparatus for supplying anesthetic agent to a fresh gas flow also includes an anesthetic agent sensor for obtaining a signal indicative of an amount of the anesthetic agent in the gas mixture including evaporated anesthetic agent, and a control unit for receiving the signal provided by the anesthetic agent sensor. The apparatus for supplying anesthetic agent to a fresh gas flow also includes a pressure sensor for obtaining a signal indicative of a pressure of the liquid chamber and providing the signal to the control unit to control the compressor to maintain the liquid pressure in the liquid chamber at a desired target pressure, and a regulation valve for regulating a flow of the liquid anesthetic agent and which regulation valve is controlled by the control unit based on at least the signal provided by the anesthetic agent sensor. The regulation valve is digitally controlled, and the control unit is configured to calculate the amount of anesthetic agent delivered based on the gas flow rate provided by the flow sensor and the agent content in the gas flow provided by the anesthetic agent sensor, and also to estimate the delivered amount of liquid based on the liquid pressure in the liquid chamber provided by the pressure sensor and the regulation valve actuation profile provided by the regulation valve.

In another embodiment a method for supplying anesthetic agent to a fresh gas flow includes collecting the liquid anesthetic agent into a liquid chamber, and applying pressure into the liquid chamber. The method for supplying anesthetic agent to a fresh gas flow also includes entering a desired target pressure into a user interface, and obtaining from a pressure sensor a signal indicative of the pressure of the liquid chamber to maintain the liquid anesthetic agent pressure at the desired target pressure. The method for supplying anesthetic agent to a fresh gas flow also includes receiving a liquid anesthetic agent in an evaporation chamber, and receiving a fresh gas in the evaporation chamber. The method for supplying anesthetic agent to a fresh gas flow also includes evaporating the liquid anesthetic agent in the evaporating chamber, and mixing the evaporated anesthetic agent with the fresh gas. The method for supplying anesthetic agent to a fresh gas flow also includes obtaining from a flow sensor a signal indicative of the gas flow either before or after mixing the fresh gas with the evaporated anesthetic agent and providing this signal to adjust the flow of the liquid anesthetic agent, and obtaining a signal indicative of an amount of the anesthetic agent in the gas mixture from an anesthetic agent sensor. The method for supplying anesthetic agent to a fresh gas flow also includes conducting the gas mixture including evaporated anesthetic agent for subject breathing and regulating by means of a regulation valve flow of the liquid anesthetic agent based on at least the signal indicative of the amount of the anesthetic agent in the gas mixture. The method for supplying anesthetic agent to a fresh gas flow also includes calculating the amount of anesthetic agent delivered based on the gas flow rate provided by the flow sensor and the agent content in the gas flow provided by the anesthetic agent sensor, and also estimating the received amount of liquid based on the liquid pressure in the liquid chamber provided by the pressure sensor and the regulation valve actuation profile provided by the regulation valve being digitally controlled.

Various other features, objects, and advantages of the invention will be made apparent to those skilled in the art from the accompanying drawings and detailed description thereof.

### BRIEF DESCRIPTION OF THE DRAWINGS

Fig. 1 illustrates an operational diagram of an arrangement for providing an inspiration gas to lungs of a subject; and
Fig. 2 presents the block diagram of an apparatus for supplying anesthethic agent to a fresh gas flow in accordance with an embodiment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

The embodiments are directed to an apparatus and a method for use in anesthesia delivery either when using intensified breathing or spontaneous breathing.

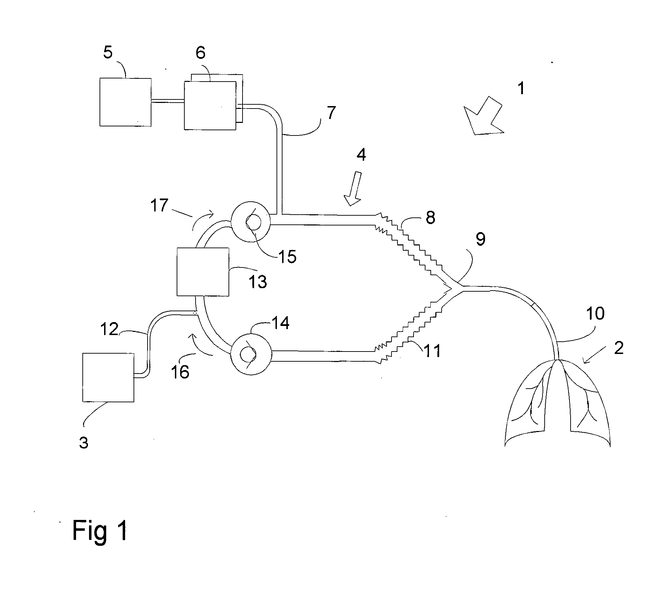

An arrangement 1 for providing an inspiration gas to lungs 2 of a subject utilizing a re-breathing system is shown in Fig. 1. The arrangement 1 comprises a ventilator 3 for assisting breathing function of the subject, a breathing circuit 4 for connecting lungs of the subject and the ventilator 3, a fresh gas mixer 5 for preparation of the appropriate breathing gas mixture of oxygen and nitrogen or nitrous oxide and to control its flow rate, and the apparatus 6 such as an evaporator for supplying anesthetic agent to the fresh gas mixture received from the fresh gas mixer and for supplying anesthetic agent mixed with the fresh gas mixture to the breathing circuit. The apparatus 6 is able to add on anesthetic agent in user dialed concentration to the fresh gas flow. For the dialing purpose there may be a user interface to receive the concentration setting. The arrangement 1 can accommodate one or more apparatus. However, according to regulations, only one of the apparatus may be selected active at a time. The complete fresh gas mixture is conducted from the apparatus to the breathing circuit 4 through the fresh gas line 7.

In the breathing circuit 4 the fresh gas coming from the fresh gas mixer 5 and the apparatus 6 is mixed with the re-circulated breathing gas at the circuit. During inspiration this mixture is guided through an inspiration line 8 to a branching unit 9 and further through a connection line 10 to subject lungs 2 expanding those. Expiration follows inspiration when the drive of the breathing gases into the lung is ceased. At this moment the compliant forces of the lungs pressurize the gas in the lungs. Expiration flow begins when the ventilator 3 opens an expiration control valve within the ventilator (not shown). Pressurized gas from the lungs 2 flows out through the connection line 10 to the branching unit and further through an expiration line 11 to a ventilator limb 12 and to the ventilator 3. Within the ventilator the exhalation gas is at least partially preserved for the next inspiration. At the time of the next inspiration, the expiration control valve of the ventilator 3 is closed, the inspiration valve of the ventilator (not shown) is opened to drive at least partly the previously exhaled breathing gas from the ventilator 3 back to the breathing circuit 4. Now the inspiration gas flows through a CO2 absorber 13 to the inspiration line where the fresh gas is added along a fresh gas line 7. An expiration valve 14 and inspiration valve 15 guide the direction of rotation of the ventilation within the breathing circuit as indicated by arrows 16 and 17.

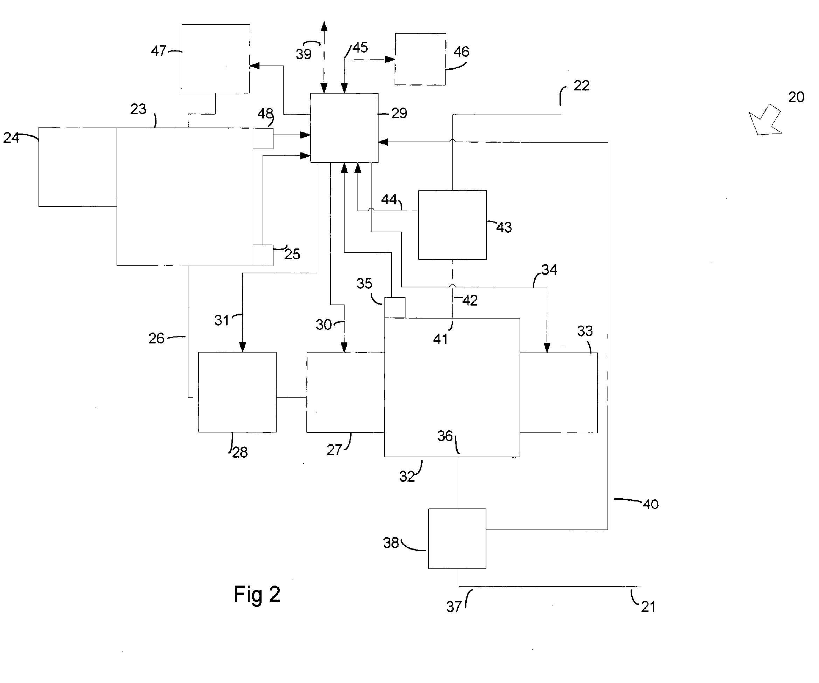

The apparatus 6 for evaporating the liquid anesthetic agent and for supplying anesthesia agent vapor to breathing gas for subject breathing presented in Fig. 2 may be a module detachable from the arrangement 1 and again remountable. The apparatus 6 comprises an inlet port 21 for the fresh gas and an outlet port 22 for the fresh gas mixed with the anesthetic agent and which outlet port is in flow communication with the fresh gas line 7 shown in Fig. 1. The apparatus 6 may also comprise a liquid chamber 23 for a liquid anesthetic agent. The liquid chamber 23 is in flow communication with an evaporation chamber 32 which is also part of the apparatus 6. The liquid chamber 23 is communicating with a filling unit 24 to fill the chamber with the liquid anesthetic. This filling unit 24 is agent specific that mates only with the transport package intended for the apparatus configuration. This allows filling only with the intended liquid. The liquid chamber 23 may have also a liquid level sensor 25 to obtain a signal indicative of a liquid level to inform e.g. the clinician of the refill need.

The liquid chamber 23 has on its lower part or preferably at the bottom an exit line 26 for the anesthetic agent liquid guiding that for the evaporation. The apparatus 6 also comprises a regulation valve 27 through which the liquid is flowing from the liquid chamber 23 and through exit line 26 to regulate the flow of the liquid anesthetic agent. This regulation valve is digitally controlled. In a proportionally controlled valve, the liquid flow could be continuous and controlled according to the demanded apparatus output A proportionally controlled valve, however, does not belong to the invention. In digital control the liquid flow is in pulses with flow rate determined by the liquid supply pressure and by varying the pulse frequency and pulse duration the total delivery is adjusted to the demanded apparatus output. The apparatus 6 may also comprise a shut-off valve 28 in the exit line 26 providing duplicate means to shut off the liquid delivery from the liquid chamber 23 if the regulation valve 27 fails open.

The apparatus 6 further comprises a control unit 29 controlling its operation. The liquid level sensor provides the signal indicative of the liquid level to the control unit which may in one way or another inform about the refill need or even automatically take care of this need. The control unit 29 may also control through a control line 31 the shut-off valve 28 to shut off the anesthetic agent liquid delivery from the liquid chamber 23 to the apparatus 6 and further may control through a control line 30 the regulation valve 27.

The liquid anesthetic is delivered from the liquid chamber 23 to an evaporation chamber 32 of the apparatus 6, where the liquid evaporates and mixes with the fresh gas flow received from the fresh gas mixer 5 through the inlet port 21. The liquid evaporation is substantially an immediate act in the evaporation chamber when the liquid is received from the liquid chamber to avoid storing the liquid in the evaporation chamber. Typically the anesthetic agent is in the liquid form in the evaporation chamber 32 only less than 1000 ms, more specifically less than 500 ms or even more specifically less than 300 ms.

The evaporation consumes energy. The fresh gas flow maximum is typically 10 L/min and the evaporated concentration at maximum 18 volume%. With these values the evaporation heat power exceeds 30 W. This power is cooling the evaporation chamber 32 and the gas within, which decreases the liquid's vapor pressure and thus reduces evaporation. To guarantee the evaporation the apparatus 6 may have a heating unit 33 to compensate with additional energy the cooling caused by evaporation. This heating energy is carried through line 34. For controlling the heat energy a temperature sensing unit 35 connected to the evaporation chamber 32 obtains a signal indicative of a temperature of the evaporation chamber 32 when evaporating the liquid anesthetic. Using the measured temperature provided by the temperature sensing unit the control unit 29 may control the energy supply to maintain optimal the temperature of the evaporation chamber 32.

As explained hereinbefore the fresh gas may be supplied from the inlet port 21 to the evaporation chamber 32 along an inlet channel 37 and through an inlet opening 36. The apparatus 6 may comprise a flow sensor 38 shown in Fig. 2 in the inlet channel 37 connected to the control unit 29 through signal line 40. Optionally the control unit 29 may get the fresh gas flow information through a communication line 39 from some other part of the apparatus 6.

From the apparatus 6 the fresh gas and evaporated anesthetic agent is guided through outlet opening 41 to outlet channel 42 guiding the flow to the outlet port 22 as shown in Fig 2 and further along the fresh gas line 7 to the breathing circuit 4 as shown in Fig. 1. An anesthetic agent sensor 43 obtaining a signal indicative of the amount of anesthetic agent at the gas flow is part of the apparatus 6 and assembled downstream the evaporation chamber 32 which sensor is in this embodiment in the outlet channel 42 or in flow communication with this outlet channel and connected through a signal line 44 to the control unit 29 providing the signal indicative of the amount of anesthetic agent at the gas flow to the control unit. This amount of anesthetic agent may be for example indicative of volumetric concentration, mass concentration or partial pressure. The anesthetic agent sensor 43 may be of any type giving information of the flowing gas concentration including infrared absorption or ultrasonic time-of-flight measurement.

The apparatus 6 may also comprise a user interface 46 for entering desired targeted amount of anesthetic agent on the apparatus 6 outlet flow for controlling the operation.. The user interface may also be common with other parts of the arrangement 1 and does not necessarily be in the same apparatus module. The user interface may also give status information of the apparatus 6 like the liquid level of the liquid chamber 23 when the refill should be made. The control unit 29 receives in the embodiment shown in Figure 2 through signal line 45 from the user interface 46 the information regarding the desired targeted amount of anesthetic agent at the apparatus output. This amount can be e.g. volumetric or mass concentration of the agent or partial pressure. Alternatively the control unit 29 may receive the value from other parts of the arrangement 1 through the communication line 39. The control unit 29 compares the signal from the anesthetic agent sensor 43 to the received desired target anesthetic agent amount and regulates the agent delivery with the regulation valve 27 to reduce the difference between the signal and the desired target anesthetic agent amount.

To ensure the liquid flow from the liquid chamber 23 along the exit line 26 to the regulation valve 27 a pressure may be applied to the chamber using a compressor 47 which may be part of the apparatus 6. This compressor pumps gas to the liquid chamber pressurizing the liquid. A signal indicative of this pressure is obtained by a pressure sensor 48 and provided to the control unit 29. Using the signal from the pressure sensor the control unit 29 controls the compressor to maintain the liquid pressure in the chamber 23 at the desired target pressure which may be entered through the user interface.

The information indicative of the fresh gas flow obtained for instance from the flow senor 38 and provided to the control unit 29 can be used to adjust the regulation valve 27 as well. The fresh gas flow can vary between 0.2 and 10 L/min and even higher up to 15 L/min. In practice step-vice fresh gas flow changes during anesthesia may be of the order of 2-4 fold, e.g. from 0.5 L/min to 1-2 L/min or vice versa. To maintain the anesthetic agent concentration requires respective 2-4 fold change in delivery rate as well. Such major step change may be a challenge for control algorithm if based only on the measured output signal from sensor 43, and even at the best the controller can respond when the output signal has already changed. Using the flow information as control unit input signals the step change already before the concentration has changed. This flow sensor can be assembled alternatively to the outlet channel 42 at the evaporation chamber 32 outlet.

As the safety-critical apparatus it has redundancy to identify problems and fail in a safe way. Critical failure that may result to erroneous delivery is a measurement error made by the anesthetic agent sensor 43. To eliminate the hazard caused by this the apparatus 6 must have independent safety system to identify the situation and respond to prevent the damage to occur. In case of stand-alone solution it is advantageous to have such protective mechanisms within the apparatus 6. In this solution the amount of anesthetic agent delivered can be calculated when knowing the gas flow rate at the outlet channel 42 and the agent content in the gas flow. For this purpose, the flow sensor 38 can be assembled instead of inlet channel 37 to outlet channel 42. As a reference, the delivered amount of liquid can be estimated from the liquid pressure in the liquid chamber 23 and the regulation valve actuation profile. In correctly working system these two agent delivery signals are substantially equal. Any mismatch in the values indicates device error that the system may respond either as messaging the user and/or shutting off the apparatus 6.

Measuring the direct agent amount output of the evaporation chamber 32 and regulating liquid anesthetic agent flow to the evaporation chamber 32 provides additional safety advantage against device failures comparing to prior art apparatus. For example, failure in sensing the liquid level of the chamber 23 in prior art apparatus may result erroneous identification of liquid in empty liquid chamber. Measuring the agent output of the evaporation chamber 32 reveals the failure immediately.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An apparatus for supplying anesthetic agent to a fresh gas flow comprising:
an evaporation chamber (32) for receiving and evaporating a liquid anesthetic agent and which evaporation chamber comprises an inlet opening (36) for a fresh gas for mixing evaporated anesthetic agent with the fresh gas and which evaporation chamber also comprises an outlet opening (41) for conducting the gas mixture including evaporated anesthetic agent out from said evaporation chamber;
a liquid chamber (23) for a liquid anesthetic agent, and which liquid anesthetic agent is received by said evaporation chamber from said liquid chamber along an exit line (26);
a compressor (47) applying a pressure to said chamber;
an anesthetic agent sensor (43) for obtaining a signal indicative of an amount of the anesthetic agent in the gas mixture including evaporated anesthetic agent;
a control unit (29) for receiving the signal provided by said anesthetic agent sensor;
a pressure sensor (48) for obtaining a signal indicative of a pressure of said liquid chamber (23) and providing said signal to said control unit to control said compressor to maintain the liquid pressure in said liquid chamber at a desired target pressure;
a regulation valve (27) for regulating a flow of the liquid anesthetic agent and which regulation valve is controlled by said control unit based on at least the signal provided by said anesthetic agent sensor; and
a flow sensor (38) for obtaining a signal indicative of the gas flow through one of said inlet opening and said outlet opening and
providing said signal to said control unit to adjust said regulation valve,
**characterized in that** said regulation valve is digitally controlled, and that said control unit (29) is configured to calculate the amount of anesthetic agent delivered based on the gas flow rate provided by said flow sensor (38) and the agent content in the gas flow provided by said anesthetic agent sensor (43), and also to estimate the delivered amount of liquid based on the liquid pressure in the liquid chamber (23) provided by said pressure sensor (48) and the regulation valve actuation profile provided by said regulation valve (27).

2. The apparatus of claim 1, further comprising a user interface (46) for entering a desired anesthetic agent amount of the gas mixture and which desired anesthetic agent amount of the gas mixture is received by said control unit (29) to control said regulation valve.

3. The apparatus of claim 1, further comprising a liquid level sensor (25) for obtaining a signal indicative of a liquid level of said liquid chamber and providing said signal to said control unit to inform about the refill need.

4. The apparatus of claim 1, further comprising a heating unit (33) to compensate said evaporation chamber with additional energy due to a cooling caused by the evaporation of the liquid anesthetic agent and also comprising a temperature sensing unit (35) obtaining a signal indicative of a temperature of said evaporation chamber and providing said signal to said control unit (29) to control the energy supply of said heating unit to maintain optimal the temperature of said evaporation chamber (32).

5. The apparatus of claim 1, further comprising a shut-off valve (28) to shut off under the control of said control unit the liquid delivery from said liquid chamber to said evaporation chamber if the regulation valve (27) fails open.

6. The apparatus of claim 1, **characterized in that** the signal indicative of an amount of the anesthetic agent in the gas mixture is the signal indicative of the anesthetic agent concentration or partial pressure in the gas mixture.

7. A method for supplying anesthetic agent to a fresh gas flow comprising:
collecting the liquid anesthetic agent into a liquid chamber (23);
applying pressure into said liquid chamber;
entering a desired target pressure into a user interface (46);
obtaining from a pressure sensor (48) a signal indicative of said pressure of said liquid chamber to maintain the liquid anesthetic agent pressure at said desired target pressure;
receiving a liquid anesthetic agent in an evaporation chamber (32);
receiving a fresh gas in said evaporation chamber;
evaporating the liquid anesthetic agent in said evaporation chamber;
mixing the evaporated anesthetic agent with the fresh gas;
obtaining from a flow sensor (38) a signal indicative of the gas flow either before or after mixing the fresh gas with the evaporated anesthetic agent and providing this signal to adjust the flow of the liquid anesthetic agent;
obtaining a signal indicative of an amount of the anesthetic agent in the gas mixture from an anesthetic agent sensor (43); and
conducting the gas mixture including evaporated anesthetic agent for subject breathing,
regulating by means of a regulation valve (27) flow of the liquid anesthetic agent based on at least the signal indicative of the amount of the anesthetic agent in the gas mixture
**characterized in that** said method also comprising calculating the amount of anesthetic agent delivered based on the gas flow rate provided by said flow sensor (38) and the agent content in the gas flow provided by said anesthetic agent sensor (43), and also estimating the received amount of liquid based on the liquid pressure in the liquid chamber (23) provided by said pressure sensor (48) and the regulation valve actuation profile provided by said regulation valve (27) being digitally controlled.

8. The method of claim 7 further comprising providing the signal indicative of the amount of the anesthetic agent in the gas mixture and the desired anesthetic agent amount of the gas mixture to a control unit (29) to control said regulating the flow.

## Patentansprüche

1. Gerät zur Zuführung von Anästhetikum zu einem Frischgasstrom, umfassend:
eine Verdampfungskammer (32) zur Aufnahme und Verdampfung eines flüssigen Anästhetikums, wobei die Verdampfungskamrrier eine Einlassöffnung (36) für ein Frischgas zwecks Mischung von verdampftem Anästhetikurn mit dem Frischgas umfasst und wobei die Verdampfungskammer auch eine Auslassöffnung (41) zur Leistung des verdampftes Anästhetikum umfassenden Gasgemischs aus der Verdampfungskammer hinaus umfasst;
eine Flüssigkeitskammer (23) für ein flüssiges Anästhetikum, wobei das flüssige Anästhetikum aus der Flüssigkeitskammer entlang einer Austrittsleitung (26) der Verdampfungskammer zugeführt wird;
einen Kompressor (47), der die Kammer mit einem Druck beaufschlagt;
einen Anästhetikumsensor (43) zum Erhalt eines Signals, das eine Menge des Anästhetikums in dem verdampftes Anästhetikum umfassenden Gasgemisch anzeigt;
eine Steuereinheit (29) zum Empfang des durch den Anästhetikumsensor bereitgestellten Signals;
einen Drucksensor (48) zum Erhalt eines Signals, das einen Druck der Flüssigkeitskammer (23) anzeigt, und zur Bereitstellung des Signals an die Steuereinheit zwecks Steuerung des Kompressors, um den Flüssigkeitsdruck in der Flüssigkeitskammer bei einem gewünschten Solldruck zu halten;
ein Regelventil (27) zur Regelung eines Stroms des flüssigen Anästhetikums, wobei das Regelventil auf der Grundlage wenigstens des durch den Anästhetikumsensor bereitgestellten Signals durch die Steuereinheit gesteuert wird; und
einen Durchflusssensor (38) zum Erhalt eines Signals, das den Gasdurchfluss durch die Einlassöffnung oder die Auslassöffnung anzeigt,
und die Bereitstellung des Signals an die Steuereinheit zwecks Einstellung des Regelventils,
**dadurch gekennzeichnet, dass** das Regelventil digital gesteuert wird und dass die Steuereinheit (29) so ausgestaltet ist,
dass sie auf der Grundlage des durch den Durchflusssensor (38) bereitgestellten Gasdurchflusses und des durch den Anästhetikumsensor (43) bereitgestellten Gehalts an Anästhetikum im Gasstrom die zugeführte Menge an Anästhetikum berechnet und dass sie auf der Grundlage des durch den Drucksensor (48) bereitgestellten Flüssigkeitsdrucks in der Flüssigkeitskammer (23) und des durch das Regelventil (27) bereitgestellten Regelventilstellprofils auch die zugeführte Flüssigkeitsmenge abschätzt.

2. Gerät nach Anspruch 1, weiterhin umfassend eine Benutzerschnittstelle (46) zur Eingabe einer gewünschten Menge an Anästhetikum im Gasgemisch, wobei die gewünschte Menge an Anästhetikum im Gasgemisch zwecks Steuerung des Regelventils der Steuereinheit (29) zugeführt wird.

3. Gerät nach Anspruch 1, weiterhin umfassend einen Flüssigkeitsstandsensor (25) zum Erhalt eines Signals, das einen Flüssigkeitsstand der Flüssigkeitskammer anzeigt, und zur Bereitstellung des Signals an die Steuereinheit zwecks Information über den Nachfüllbedarf.

4. Gerät nach Anspruch 1, weiterhin umfassend eine Heizeinheit (33) zur ausgleichenden Versorgung der Verdampfungskammer mit zusätzlicher Energie aufgrund einer durch die Verdampfung des flüssigen Anästhetikums bedingten Kühlung und auch umfassend eine Temperaturfühleinheit (35), die ein Signal erhält, das eine Temperatur der Verdampfungskammer anzeigt und das Signal an die Steuereinheit (29) bereitstellt, um die Energieversorgung der Heizeinheit zu steuern und so die Temperatur der Verdampfungskammer (32) optimal zu halten.

5. Gerät nach Anspruch 1, weiterhin umfassend ein Absperrventil (28), um im Rahmen der Steuerung der Steuereinheit die Flüssigkeitszufuhr von der Flüssigkeitskammer zur Verdampfungskammer abzusperren, wenn sich das Regelventil (27) nicht öffnet.

6. Gerät nach Anspruch 1, **dadurch gekennzeichnet, dass** das eine Menge des Anästhetikums im Gasgemisch anzeigende Signal das die Konzentration oder den Partialdruck des Anästhetikums im Gasgemisch anzeigende Signal ist.

7. Verfahren zur Zuführung von Anästhetikum zu einem Frischgasstrom, umfassend:
Sammeln des flüssigen Anästhetikums in einer Flüssigkeitskammer (23);
Beaufschlagen der Flüssigkeitskammer mit Druck;
Eingeben eines gewünschten Solldrucks in eine Benutzerschnittstelle (46);
Erhalten eines Signals von einem Drucksensor (48), das den Druck der Flüssigkeitskammer anzeigt, um den Druck des flüssigen Anästhetikums bei dem gewünschten Solldruck zu halten;
Aufnehmen eines flüssigen Anästhetikums in einer Verdampfungskammer (32);
Aufnehmen eines Frischgases in der Verdampfungskammer;
Verdampfen des flüssigen Anästhetikums in der Verdampfungskammer;
Mischen des verdampften Anästhetikums mit dem Frischgas;
Erhalten eines Signals von einem Durchflusssensor (38), das den Gasdurchfluss vor oder nach der Mischung des Frischgases mit dem verdampften Anästhetikum anzeigt, und Bereitstellen dieses Signals zwecks Einstellung des Stroms des flüssigen Anästhetikums;
Erhalten eines Signals, das eine Menge des Anästhetikums im Gasgemisch anzeigt, von einem Anästhetikumsensor (43); und
Leisten des verdampftes Anästhetikum umfassenden Gasgemischs zwecks Beatmung einer Person,
Regeln des Stroms des flüssigen Anästhetikums auf der Grundlage wenigstens des Signals, das die Menge des Anästhetikums im Gasgemisch anzeigt, mittels eines Regelventils (27),
**dadurch gekennzeichnet, dass** das Verfahren auch umfasst: die Berechnung der Menge an zugeführtem Anästhetikum auf der Grundlage des durch den Durchflusssensor (38) bereitgestellten Gasdurchflusses und des durch den Anästhetikumsensor (43) bereitgestellten Gehalts an Anästhetikum im Gasstrom und auch die Abschätzung der aufgenommenen Flüssigkeitsmenge auf der Grundlage des durch den Drucksensor (48) bereitgestellten Flüssigkeitsdrucks in der Flüssigkeitskammer (23) und des durch das digital gesteuerte Regelventil (27) bereitgestellten Regelventilstellprofils.

8. Verfahren nach Anspruch 7, weiterhin umfassend die Bereitstellung des Signals, das die Menge des Anästhetikums im Gasgemisch anzeigt, und der gewünschten Menge an Anästhetikum im Gasgemisch an eine Steuereinheit (29) zwecks Steuerung der Regelung des Stroms.

## Revendications

1. Appareil pour alimenter en un agent anesthésique un flux de gaz frais, ledit appareil comprenant :
une chambre d'évaporation (32) destinée à recevoir et
faire s'évaporer un agent anesthésique liquide, ladite chambre d'évaporation comprenant une ouverture d'entrée (36) pour un gaz frais pour mélanger l'agent anesthésique évaporé et le gaz frais, ladite chambre d'évaporation comprenant en outre une ouverture de sortie (41) pour acheminer ledit mélange de gaz comprenant l'agent anesthésique évaporé hors de ladite chambre d'évaporation ;
une chambre pour liquide (23) pour un agent anesthésique liquide, ledit agent anesthésique liquide étant reçu par ladite chambre d'évaporation depuis ladite chambre pour liquide le long d'une conduite de sortie (26) ;
un compresseur (47) appliquant une pression à ladite chambre ;
un capteur d'agent anesthésique (43) pour obtenir un signal indiquant une quantité d'agent anesthésique dans le mélange de gaz comprenant l'agent anesthésique évaporé ;
une unité de commande (29) pour recevoir le signal fourni par ledit capteur d'agent anesthésique ;
un capteur de pression (48) pour obtenir un signal indiquant une pression de ladite chambre pour liquide (23) et pour fournir ledit signal à ladite unité de commande pour commander au dit compresseur de maintenir la pression de liquide dans ladite chambre pour liquide à une pression cible souhaitée ;
une vanne de régulation (27) pour réguler un flux de l'agent anesthésique liquide, ladite vanne de régulation étant commandée par ladite unité de commande sur la base au moins du signal fourni par ledit capteur d'agent anesthésique ; et
un capteur de flux (38) pour obtenir un signal indiquant le flux de gaz à travers l'une de ladite ouverture d'entrée et de ladite ouverture de sortie et
fournir au dit signal à ladite unité de commande pour ajuster ladite vanne de régulation,
**caractérisé en ce que** ladite vanne de régulation est à commande numérique, et **en ce que** ladite unité de commande (29) est configurée pour calculer la quantité d'agent anesthésique délivrée sur la base du flux de gaz fourni par ledit capteur de flux (38) et la teneur en agent dans le flux de gaz fournie par ledit capteur d'agent anesthésique (43), et également pour estimer la quantité de liquide délivrée sur la base de la pression de liquide dans la chambre pour liquide (23) fournie par ledit capteur de pression (48) et le profil d'actionnement de la vanne de régulation fourni par ladite vanne de régulation (27).

2. Appareil selon la revendication 1, comprenant en outre une interface utilisateur (46) pour entrer une quantité d'agent anesthésique souhaitée dans le mélange de gaz et ladite quantité d'agent anesthésique souhaitée dans le mélange de gaz est reçue par ladite unité de commande (29) pour commander ladite vanne de régulation.

3. Appareil selon la revendication 1, comprenant en outre un capteur de niveau de liquide (25) pour obtenir un signal indiquant un niveau de liquide de ladite chambre pour liquide et fournir ledit signal à ladite unité de commande pour informer du besoin de remplissage.

4. Appareil selon la revendication 1, comprenant en outre une unité de chauffage (33) pour compenser le refroidissement provoqué par l'évaporation de l'agent anesthésique liquide de ladite chambre d'évaporation avec de l'énergie supplémentaire et comprenant en outre une unité capteur de température (35) obtenant un signal indiquant une température de ladite chambre d'évaporation et fournissant ledit signal à ladite unité de commande (29) pour commander à la source d'énergie de ladite unité de chauffage de maintenir optimale la température de ladite chambre d'évaporation (32).

5. Appareil selon la revendication 1, comprenant en outre une vanne d'arrêt (28) pour arrêter, sous la commande de ladite unité de commande, la livraison de liquide de ladite chambre pour liquide à ladite chambre d'évaporation si la vanne de régulation (27) ne s'ouvre pas.

6. Appareil selon la revendication 1, **caractérisé en ce que** le signal indiquant une quantité de l'agent anesthésique dans le mélange de gaz est le signal indiquant la concentration en agent anesthésique ou la pression partielle dans le mélange de gaz.

7. Procédé pour alimenter en un agent anesthésique un flux de gaz frais, ledit procédé comprenant :
la collecte de l'agent anesthésique liquide dans une chambre pour liquide (23) ;
l'application de pression dans ladite chambre pour liquide ;
l'entrée d'une pression cible souhaitée dans une interface utilisateur (46) ;
l'obtention, d'un capteur de pression (48), d'un signal indiquant ladite pression de ladite chambre pour liquide pour maintenir la pression d'agent anesthésique liquide à ladite pression cible souhaitée ;
la réception d'un agent anesthésique liquide dans une chambre d'évaporation (32) ;
la réception d'un gaz frais dans ladite chambre d'évaporation ;
l'évaporation de l'agent anesthésique liquide dans ladite chambre d'évaporation ;
le mélange de l'agent anesthésique évaporé avec le gaz frais ;
l'obtention, d'un capteur de flux (38), d'un signal indiquant le flux de gaz avant ou après le mélange du gaz frais avec l'agent anesthésique évaporé et la fourniture de ce signal pour ajuster le flux de l'agent anesthésique liquide ;
l'obtention d'un signal indiquant une quantité de l'agent anesthésique dans le mélange de gaz d'un capteur d'agent anesthésique (43) ; et
l'acheminement du mélange de gaz comprenant l'agent anesthésique évaporé pour la respiration d'un sujet,
la régulation, au moyen d'une vanne de régulation (27),
du flux d'agent anesthésique liquide sur la base au moins du signal indiquant la quantité de l'agent anesthésique dans le mélange de gaz,
**caractérisé en ce que** ledit procédé comprend également le calcul de la quantité d'agent anesthésique délivré sur la base du flux de gaz fourni par ledit capteur de flux (38) et la teneur en agent dans le flux de gaz fournie par ledit capteur d'agent anesthésique (43),
ainsi que l'estimation de la quantité de liquide reçue sur la base de la pression liquide dans la chambre pour liquide (23) fournie par ledit capteur de pression (48) et le profil d'actionnement de la vanne de régulation fourni par ladite vanne de régulation (27) à commande numérique.

8. Procédé selon la revendication 7, comprenant en outre la fourniture du signal indiquant la quantité de l'agent anesthésique présente dans le mélange de gaz et la quantité souhaitée d'agent anesthésique dans le mélange de gaz à une unité de commande (29) pour commander ladite régulation du flux.
